# EUROPEAN PATENT APPLICATION

(11) **EP 4 663 092 A1**
(43) Date of publication of application: **17.12.2025**
(21) Application number: 23916939.4
(22) Date of filing: 21.04.2023
(51) Int. Cl.: A61B 1/005, A61B 1/008

(54) **ENDOSCOPE AND TIP BENDING SECTION OF ENDOSCOPE**

(71) Applicant: Macrolux Medical Technology Co., Ltd., Shenzhen, Guangdong 518132 (CN)
(72) Inventor: WANG, Zhe, Shenzhen, Guangdong 518132 (CN); WANG, Jin, Shenzhen, Guangdong 518132 (CN); WU, Wenhao, Shenzhen, Guangdong 518132 (CN)
(74) Representative: Manitz Finsterwald Patent- und Rechtsanwaltspartnerschaft mbB
(86) International application number: PCT/CN2023/089920
(87) International publication number: WO 2024/152466

(57) **Abstract**

A tip bending section (21) of an endoscope, which relates to the field of endoscopes. The tip bending section (21) of the endoscope comprises at least two unit segments; and a connection body (214), the connection body (214) being located between two adjacent unit segments, the connection body (214) being fixedly connected to two adjacent unit segments and bendably deformable, and the bendably deformable connection body (214) being used to enable the two adjacent unit segments to undergo relative deviation; the material of the connection body (214) differs from at least one adjacent unit segment, and the material of the connection body (214) enables the bending fatigue resistance of the connection body (214) to be higher than that of an adjacent unit segment of a different material; and/or the connection body (214) has a reinforcement structure, and the reinforcement structure is used to improve the bending fatigue resistance of the connection body (214). The present invention is able to solve the problem where tip bending sections (21) of endoscopes are prone to damage after being repeatedly bent.

## Description

### TECHNICAL FIELD

The present disclosure relates to endoscopes, in particular to tip bending sections of endoscopes.

### BACKGROUND OF THE INVENTION

An endoscope is a medical device used commonly. A typical structure of an endoscope may include a handle, an insertion tube and a tip. The handle can be grasped and operated by an operator, and may be often equipped with an imaging display unit or an interface connected with an external display. The insertion tube may be connected to the distal end of the handle and may be used for inserting into an organism. The tip may be positioned at the terminal of the distal end of the endoscope, and may usually include a visual inspection unit (like a camera) and an illuminating unit (such as a LED or an optical fiber outlet) for illuminating and imaging the internal tissue of the organism.

The insertion tube may generally include an extension section and a tip bending section arranged between the extension section and the tip. The length of the tip bending section may often be much smaller than that of the extension section. The tip bending section can be bent under the control of the handle, so as to control the direction of the insertion tube impelled in the organism. During the use of the endoscope, the insertion tube can transmit longitudinal thrust and rotary torque applied at the handle to the distal end of the endoscope. In addition, the insertion tube may be provided inside with a channel that can allow the cables of the visual inspection unit and the illuminating unit, as well as some conduit devices, to pass through.

The tip bending section of the endoscope may generally include a distal-end segment, a proximal-end segment, and a plurality of identical or similar intermediate segments. The distal-end segment may be used to connect with the tip, and the proximal-end segment may be used to connect with the extension section of the insertion tube. In order to bend the tip bending section, each segment of the tip bending section may need to be able to swing relatively; in this regard, the tip bending section may often be of an articulated structure, in which the articulation may be achieved by connecting each segment with each other via rivets (for example, a structure disclosed in the Chinese utility model patent CN209059122U or CN208582372U), or via a hinge shaft integrally arranged with each segment (such as a structure disclosed in the Chinese utility model patent CN204192566U). However, the assembly of such structures is complex and the production cost is high.

In order to solve the above problems, there may also be some tip bending sections that are of a molded single-piece structure. Each segment of the molded single-piece structure may be of an integrated structure, and local connection may be formed between adjacent segments via a connecting piece. The connecting piece may be relatively weak in strength, and can be bent and deformed under an external force, achieves the bending of the tip bending section. However, the connecting piece is prone to damage after repeated bending in practice, also leading to a low service life of the insertion tube. Additionally, there may also be limited to the torsional stiffness and longitudinal stiffness of the tip bending section caused by the connecting piece, and it may be uneasy to guarantee the stability of the structure.

### SUMMARY OF THE INVENTION

### Technical Problem

A main technical problem solved by the present disclosure may be that the tip bending sections in the existing endoscopes are prone to damage after repeated bending.

### Technical Solutions

According to a first aspect of the present disclosure, a tip bending section of an endoscope is provided.

The tip bending section of an endoscope may include:
at least two segments; and
a connecting member arranged between two adjacent segments and fixedly connected with the two adjacent segments, the connecting member being capable of being bent and deformed to allow the two adjacent segments to be deflected relative to each other;
the material of the connecting member being different from that of at least one of the two adjacent segments so that the bending fatigue-resistant strength of the connecting member is higher than that of the other adjacent segment with different material; and/or, the connecting member having a reinforcing structure for improving the bending fatigue-resistant strength of the connecting member.

In some embodiments of the present disclosure, the tip bending section may include a structure reinforcing piece that may form the structure reinforcing piece and may be fixedly engaged with the connecting member.

In some embodiments of the present disclosure, the structure reinforcing piece and the segments may be formed integrally; or, the structure reinforcing piece may be a molded piece and may be fixedly assembled with the segments.

In some embodiments of the present disclosure, the structure reinforcing piece may have a lengthwise direction consistent with the axial direction of the tip bending section.

In some embodiments of the present disclosure, the connecting member between every two segments of at least three adjacent segments may be arranged along the extension axis of the tip bending section, the three adjacent segments may form a codirectional bending section with the same deflection direction, and the structure reinforcing piece on the codirectional bending section may be in a run-through manner on every segment of the codirectional bending section.

In some embodiments of the present disclosure, the structure reinforcing piece may be made of metal, fiber or resin.

In some embodiments of the present disclosure, the material of the structure reinforcing piece may be one of stainless steel, tungsten, cotton, linen, nylon and PP.

In some embodiments of the present disclosure, the structure reinforcing piece may be in a form of a single strand or more than two strands.

In some embodiments of the present disclosure, the connecting member may be provided with a concave portion arranged on the inner side and/or outer side of the tip bending section and configured to make the thickness of the connecting member thinner.

In some embodiments of the present disclosure, the structure reinforcing piece may be exposed in the concave portion and be fixedly engaged with the bottom wall of the concave portion.

In some embodiments of the present disclosure, the structure reinforcing piece and the connecting member may be engaged everywhere along the extension axis of the tip bending section.

In some embodiments of the present disclosure, each segment may be an integral injection molded part, and the structure reinforcing piece may be an injection molded embedded piece wrapped in the integral injection molded part.

In some embodiments of the present disclosure, the structure reinforcing piece may be bonded and fixed with the segment and the connecting member.

According to a second aspect of the present disclosure, an endoscope may be provided.

The endoscope may include:
a handle for grasping and operating by an operator;
a tip arranged on a terminal of a distal end of the endoscope for illumination and/or imaging; and
an insertion tube connected at the distal end of the handle for inserting into an organism, the insertion tube including an extension section and a tip bending section arranged between the extension section and the tip, the tip bending section being capable of being bent under the control of the handle so as to be capable of controlling a direction of the insertion tube impelled in the organism;
wherein the tip bending section of the endoscope may include:
   at least two segments; and
   a connecting member arranged between two adjacent segments and fixedly connected with the two adjacent segments, the connecting member being capable of being bent and deformed to allow the two adjacent segments to deflect relative to each other;
   the material of the connecting member being different from that of at least one of the two adjacent segments to allow the bending fatigue-resistant strength of the connecting member to be higher than that of the segment with different material; and/or, the connecting member having a reinforcing structure for improving the bending fatigue-resistant strength of the connecting member.

In some embodiments of the present disclosure, the tip bending section may include a structure reinforcing piece, the reinforcing structure formed by the structure reinforcing piece, and the structure reinforcing piece may be fixedly engaged with the connecting member.

In some embodiments of the present disclosure, the structure reinforcing piece and the segment may be formed integrally, or, the structure reinforcing piece may be a molded piece and may be fixedly assembled with the segment.

In some embodiments of the present disclosure, the direction of the length of the structure reinforcing piece may be in line with the axial direction of the tip bending section.

In some embodiments of the present disclosure, the connecting member between every two segments of at least three adjacent segments may be arranged along the extension axis of the tip bending section, the at least three adjacent segments may form a codirectional bending section with the same deflection direction, and the structure reinforcing piece on the codirectional bending section may be run through the segments on the codirectional bending section.

In some embodiments of the present disclosure, the structure reinforcing piece may be made of metal, fiber or resin.

In some embodiments of the present disclosure, the material of the structure reinforcing piece may be one of stainless steel, tungsten, cotton, linen, nylon and PP.

In some embodiments of the present disclosure, the structure reinforcing piece may be in a form of a single strand or more than two strands.

In some embodiments of the present disclosure, the connecting member may be provided with a concave portion arranged on the inner side and/or outer side of the tip bending section and configured to make the thickness of the connecting member thinner.

In some embodiments of the present disclosure, the structure reinforcing piece may be exposed in the concave portion and be fixedly engaged with the bottom wall of the concave portion.

In some embodiments of the present disclosure, the structure reinforcing piece and the connecting member may be engaged everywhere along the extension axis of the tip bending section.

In some embodiments of the present disclosure, each segment may be an integral injection molded part, and the structure reinforcing piece may be an injection molded embedded piece wrapped in the integral injection molded part.

In some embodiments of the present disclosure, the structure reinforcing piece may be bonded and fixed with the segment and the connecting member.

### Beneficial Effects

With the technical solutions mentioned above, the connecting member may use different material so that the bending fatigue-resistant strength of the connecting member can be higher than that of the adjacent segment; and/or, since the tip bending section may be provided with a structure reinforcing piece and the connecting member may have a reinforcing structure for improving the bending fatigue-resistant strength of the connecting member, during repeated deflection caused by bending and deforming of two connecting members between adjacent two segments, the connecting member for deflecting and hinging two adjacent segments can have a higher service life, which is conducive to avoiding the failure of the parts corresponding to the connecting member in the tip bending section, and thus is conducive to improving the service life of the endoscope.

### BRIEF DESCRIPTION OF THE DRAWINGS

FIG. 1 is a schematic structure view of an endoscope that has a tip bending section corresponding to the one in some embodiments of the present disclosure;
FIG. 2 is a perspective view of the tip bending section shown in FIG. 1;
FIG. 3 is a side view, of the tip bending section shown in FIG. 2, viewed from the distal end towards the proximal end along the axis of the tip bending section;
FIG. 4 is a section view of FIG. 3 along A-A;
FIG. 5 is a partial enlarged view at B in FIG. 4; and
FIG. 6 is a partial enlarged view of a portion of the tip bending section of an endoscope in other embodiments, the portion corresponding to that shown in FIG. 5.

### LIST OF REFERENCE NUMERALS

- 10: handle;
- 20: insertion tube;
- 21: tip bending section;
- 211: distal-end segment;
- 212: intermediate segment;
- 213: proximal-end segment;
- 214: connecting member;
- 215: concave portion;
- 22: extension section;
- 23: rope perforation;
- 24: structure reinforcing piece;
- 25: working channel;
- 30: tip.

### DETAILED DESCRIPTION

The present disclosure is further described in detail below through specific embodiments in combination with the drawings, wherein, similar elements in different embodiments adopt associated similar element labels. In the following embodiments, many details are described in order to make the application be better understood. However, those skilled in the art can easily realize that some features can be omitted in different cases or can be replaced by other elements, materials and methods. In some cases, some operations related to the present disclosure are not shown or described in the specification in order to avoid the core part of the present disclosure being overwhelmed by excessive descriptions, and for those skilled in the art, it is not necessary to describe these relevant operations in detail, they can completely understand the relevant operations according to the description in the specification and the general technical knowledge of the field.

In addition, the features, operations or characteristics described in the specification may be combined in any appropriate manner to form various embodiments. At the same time, the steps or actions described in the method may be sequenced or adjusted in a manner apparent to those skilled in the art. Therefore, the sequences in the specification and the drawings are intended to clearly describe an embodiment and are not meant to be a required sequence unless it is indicated otherwise that a sequence must be followed.

The serial numbers assigned to the parts in the present disclosure, such as "first", "second", etc., are only used to distinguish the described objects, and do not have any sequential or technical meaning. The terms "connect" and "couple" as mentioned in the present disclosure, unless otherwise specified, include direct and indirect connection (coupling).

In the present disclosure, connecting members 214 on a tip bending section 21 of an endoscope used to form a hinge may be made of materials with higher bending fatigue-resistant strength, or may be provided with a reinforcing structure, which can improve the bending fatigue-resistant strength of the connecting members 214, helping to avoid damage after repeated bending of the tip bending section 21.

Embodiments of the tip bending section of an endoscope in the present disclosure are described as follows.

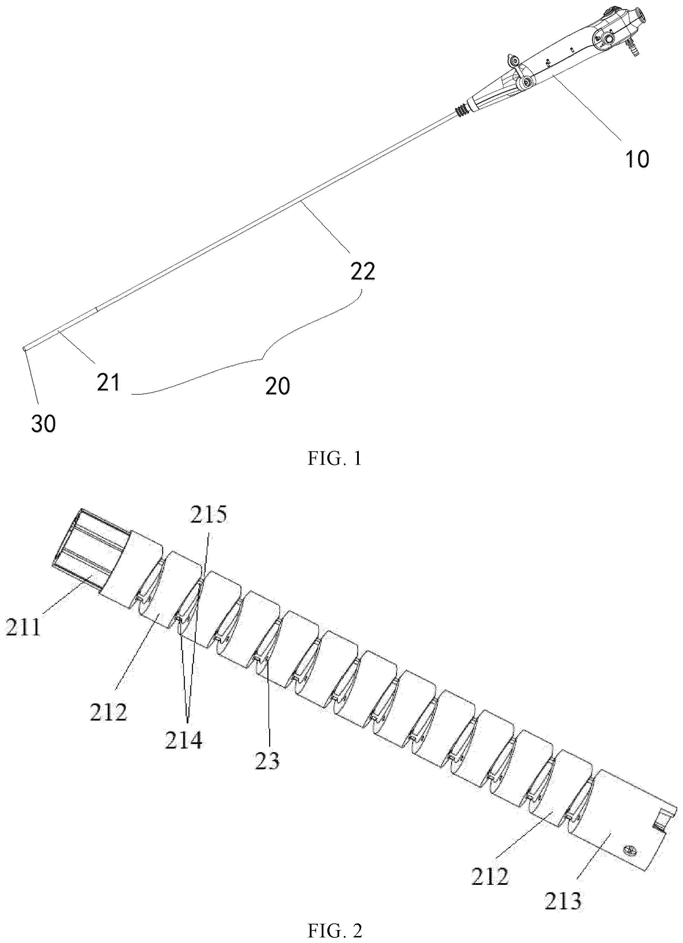

Please refer to FIG. 1, in some embodiments of the present disclosure, the tip bending section 21 of an endoscope may be arranged on an insertion tube 20 and connected between a tip 30 and an extension section 22 of the insertion tube 20 of the endoscope. The proximal end of the tip bending section 21 may be connected with the distal end of the extension section 22, and the distal end of the tip bending section 21 may be connected with the tip 30. The tip bending section 21 may often be smaller than the extension section 22 in length, and may be bent under the control of a handle 10 so that the direction of the insertion tube 20 impelled in an organism can be controlled. The proximal end of the insertion tube 20 may be fixed on the handle 10, allowing transmitting longitudinal thrust and rotary torque applied to the handle 10 via the extension section 22 and the tip bending section 21.

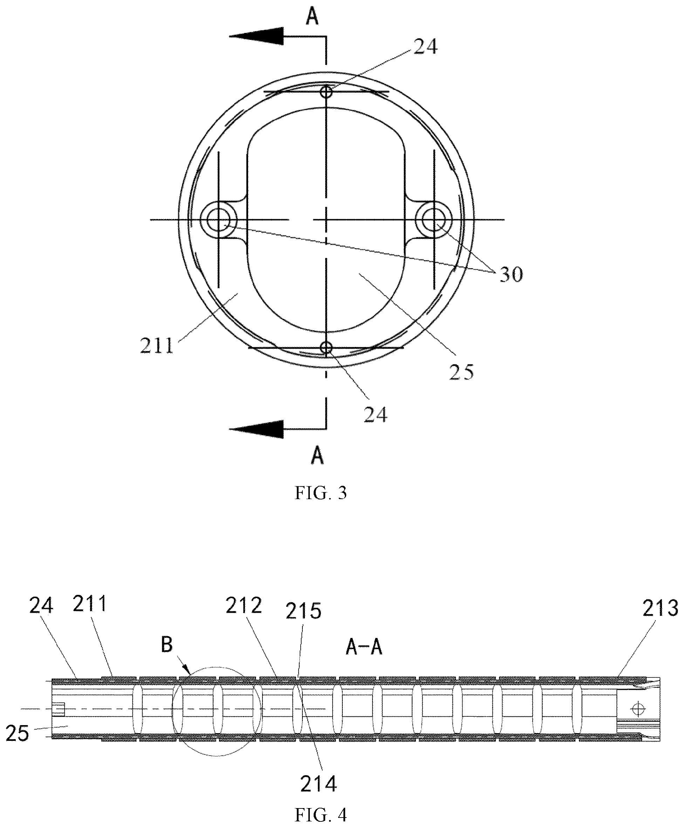

Please refer to FIG. 2 and FIG. 4, in some preferred embodiments, the tip bending section 21 of the endoscope may include multiple segments, namely, a distal-end segment 211, an intermediate segment 212 and a proximal-end segment 213, which are arranged from the distal end towards the proximal end of the tip bending section 21. There may be only one distal-end segment 211 and only one proximal-end segment 213, which are connected to the tip 30 and the extension section 22 of the endoscope, respectively. There may be a plurality of intermediate segments 212 to adjust the bending of the tip bending section 21. In other embodiments, the number of the intermediate segments 212 may also be increased or decreased as needed. Those skilled in the art know that, as shown in FIG. 3 and FIG. 4, there may generally be a working channel provided inside the tip bending section 21 to allow a cable of an visual inspection unit and that of an illuminating unit, as well as some conduit devices, to pass through. In addition, a protective cover may be provided to cover the exterior of the tip bending section 21 of the endoscope to isolate the tip bending section 21 from external environment.

The tip bending section 21 may further include connecting members 214. Each segment of the tip bending section 21 may be connected by two connecting members 214. The two connecting members 214 may be connected to the outer wall of a corresponding segment, and may be arranged respectively on the opposite sides of the tip bending section 21 in a radial direction, that is, 180 degrees apart in the circumferential direction of the tip bending section. The circumferential direction refers to a direction around the extension axis of the tip bending section 21. The connecting member 214 may be bent and deformed to make two adjacent segments deflect relative to each other. As those skilled in the art know, provision of the two connecting members 214 is to determine the deflection axis of the segments, which may be considered as an axis of swing caused by relative deflection of adjacent two segments; and the deflection axis may be determined by the circumferential position of the connecting members 214, be coincided with a connecting line of the two connecting members 214, and be perpendicular to the extension axis of the tip bending section 21. **In** other embodiments, the number of the connecting members between adjacent two segments may also be more than two or less than two. For example, there may be only one connecting member; in this regard, the width of the connecting member (i.e. a size in a direction perpendicular to the extension axis of the tip bending section) may be designed to be large so as to meet the stability of the entire tip bending section . For another example, there may be three or four connecting members arranged in a straight line. In addition, when there are more than two connecting members, the widths of the connecting members may be the same or different.

In some preferred embodiments, the tip bending section 21 may be molded integrally by injection molding to form a molded single-piece structure; and the connecting members 214 may be the ones in the tip bending section 21 for connecting two adjacent segments. In other embodiments, the segments and the connecting members 214 may be manufactured separately, and then assembled into a whole, such as bonding and fixing into a whole, or fixing into a whole by a way like ultrasonic welding; in this regard, a related structure reinforcing piece is preferably a molded piece. In still other embodiments, each connecting member 214 may be formed integrally on one of its adjacent segments and then connected with the other segment to form a whole. In some preferred embodiments, the material of the tip bending section 21 may be plastic, rubber, etc., and those skilled in the art may select an appropriate material according to needs.

In some embodiments, the connecting members 214 may be arranged along the extension axis of the tip bending section 21, each segment may form a codirectional bending section deflected in the same direction, the deflection axis of each segment may be parallel to each other, and the tip bending section 21 can only swing back and forth in two opposite directions. Each segment may be positioned in a same codirectional bending section to facilitate the manufacturing and control of the tip bending section 21. In other embodiments, for the tip bending section 21 that needs to be bent in four directions, the connecting members 214 on both axial sides of the segment arranged at the middle of three adjacent segments can have a 90 degree position difference in the circumferential direction, and cross swinging motion of the axis of swing can be achieved by means of the two connecting members 214 on both axial sides of the segment.

Endoscopes can be classified into disposable endoscopes and reusable endoscopes. No matter what kind of an endoscope, in order to avoid the failure of the connecting members 214 due to repeated bending during the use of the endoscope, the tip bending section 21 of the endoscope may also include structure reinforcing pieces 24, which may form a composite structure as a whole together with the connecting members 214 as well as adjacent segments. The structure reinforcing pieces 24 may allow the connecting members 214 to have a reinforcing structure that can improve the bending fatigue-resistant strength of the connecting members 214. The structure reinforcing pieces 24 may have a lengthwise direction consistent with a direction in which the tip bending section 21 is extended. The structure reinforcing pieces 24 may be fixedly engaged with the connecting members 214 and adjacent two segments, thereby improving the bending fatigue-resistant strength of the connecting members 214. The fixed engagement among the structure reinforcing pieces 24, the connecting members 214 and adjacent segments may mean that each part of each structure reinforcing piece 24 is fixed with each connecting member 214 and adjacent segments, or, each structure reinforcing piece 24 is fixed with each connecting member 214 and adjacent segments by a plurality of fixed points, which can strengthen the connecting members 214 in structure.

In some preferred embodiments, the tip bending section 21 is an integral injection molded part produced by injection molding process using a mold, the structure reinforcing piece 24 may be wrapped in the integral injection molded part in the form of injection molded embedded piece, which can easily manufacture the tip bending section 21 efficiently. The structure reinforcing piece 24 may be in the form of injection molded embedded piece, and may also be engaged and fixed with segments along the extension axis of the tip bending section 21 to form a composite structure similar to composite materials, which is conducive to obtaining higher bending fatigue-resistant strength. In other embodiments, a number of fixed points may also be provided between the structure reinforcing piece 24 and the connecting member 214, and between the structure reinforcing piece 24 and the segments. The fixed points may be extended along the lengthwise direction of the structure reinforcing piece 24, which can also improve the bending fatigue-resistant strength of the connecting member 214. In other embodiments, the structure reinforcing pieces 24 may also be manufactured separately, then assembled and fixedly engaged with the connecting members 214 and the segments. For example, the connecting members 214 and the segments may be provided with perforations through which the structure reinforcing pieces 24 may pass to bond and fix with the connecting members 214 and the segments. For another example, it may be fixed with the connecting members 214 and the segments by means of hot melting, friction welding, ultrasonic welding, etc..

In some preferred embodiments, the structure reinforcing pieces 24 may be made of metal, fiber or resin. When metal is used, stainless steel tungsten and so on may be preferred. When fiber is used, cotton, hemp and so on may be preferred. When resin is used, nylon, PP and so on may be preferred.

In some preferred embodiments, the structure reinforcing pieces 24 may be elastic to allow elastic bending, and the elasticity of the structure reinforcing pieces 24 may be greater than that of the connecting members 214. **In** some embodiments, the structure reinforcing pieces 24 may be made of elastic metal materials, which can achieve high flexibility, making the flexibility of the structure reinforcing pieces 24 much higher than that of the connecting members 214. When in use, compared with the tip bending section 21 without the structure reinforcing pieces 24, the tip bending section 21 with the structure reinforcing pieces 24 can have higher bending fatigue-resistant strength, thus obtaining higher service life.

The structure reinforcing pieces 24 may be in a form of a single strand or more than two strands. For example, when the structure reinforcing pieces 24 are made of metal, one metal wire (such as stainless steel wire) or more than two metal wires (such as stainless steel wire rope) may be used. For another example, they are made of fiber material, the fiber material may be a single strand or a plurality of strands. In other embodiments, the structure reinforcing pieces 24 may be of a mesh structure (that is, more than two strands of wire intersected). Preferably, the extension axis of the tip bending section may pass through a mesh surface formed by the structure reinforcing pieces 24 of the mesh structure. In this regard, in order to make the structure reinforcing pieces 24 and segments well engaged with the connecting members 214, preferably, the structure reinforcing pieces 24 may be an injection molded embedded piece wrapped in the integral injection molded part.

In some preferred embodiments, the structure reinforcing pieces 24 on the codirectional bending section may connect with each segment on the codirectional bending section in a run-through manner, which is simple in structure and easy to manufacture.

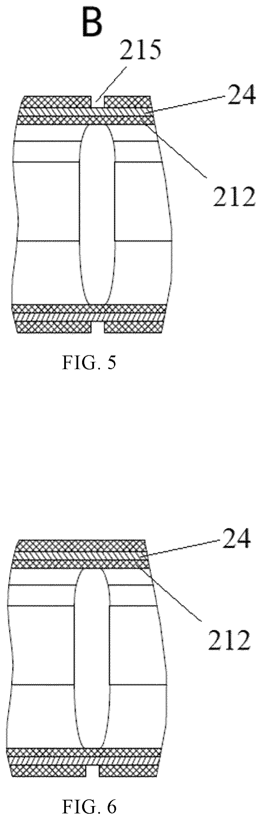

In some preferred embodiments, the connecting member 214 may be provided with a concave portion 215 arranged on the inner wall and/or outer wall of the tip bending section 21 for making the thickness of the connecting member 214 thinner. In some embodiments, as shown in FIG. 4 and FIG. 5, the concave portion 215 is arranged on the outer wall of the tip bending section 21, allowing the thickness size of the connecting member 214 along the radial direction of the insertion tube 20 to be smaller. Accordingly, it is more conducive to the bending and deforming of the tip bending section, and the bending fatigue-resistant strength of the connecting member 214 can be improved by the structure reinforcing piece 24. Preferably, the structure reinforcing piece 24 may be exposed in the concave portion 215 and fixedly engaged with the bottom wall of the concave portion 215, so as to observe the assembly and working state of the structure reinforcing piece 24.

In other embodiments, as shown in FIG. 6, the structure reinforcing piece 24 may also be wrapped completely by the segment and the connecting member 214. In this regard, the structure reinforcing piece 24 cannot be seen from the outside of the tip bending section 21. In addition, the concave portion 215 on the connecting member 214 may also be arranged on the inner wall of the tip bending section 21.

Two rope perforations 23 may be arranged on the segment to control the bending of the tip bending section 21. The connecting line of the two rope perforations 23 may be perpendicular to the connecting line of the connecting members 214 on both sides. The two rope perforations 23 may each be used for a pulling rope to move through respectively. The distal end of the pulling rope may be fixed on the distal-end segment 211, and the proximal end of the pulling rope may be connected to a drive unit inside the handle 10. Under the control of the drive unit, one pulling rope may be tensioned and the other pulling rope may be loosened, so that the tip bending section 21 can be bent towards a side where the pulling rope is tensioned. The pulling rope may be a steel wire rope, and the drive unit may be controlled manually or automatically.

During the use of the endoscope, the handle 10 may be held by an operator to transmit to the insertion tube 20 the longitudinal thrust along a direction in which the axis of the insertion tube 20 is extended and the rotary torque around the axis of the insertion tube 20, and after the insertion tube 20, the tip bending section 21 may be bent with the control of the handle 10, so that the tip 30 at the uttermost distal end of the insertion tube 20 can be moved to a target position. When the tip bending section 21 is repeatedly bent, the structure reinforcing piece 24 can strengthen the connecting member 214 between two adjacent segments, thus enhancing the bending fatigue-resistant strength of the connecting member 214, avoiding the failure of the connecting member 214 and improving the service life of the tip bending section 21. Meanwhile, since the structure of the tip bending section 21 is strengthened, the torsional and longitudinal stiffness can be also better, and the maneuverability can be better. Compared with fabricated articulated structures, it is more convenient to manufacture with lower cost; and since there is no need to provide rivets, articulated shafts and other structures, it is also possible to obtain larger internal space as the working channel 25.

In the above embodiments, the bending fatigue-resistant strength of the tip bending section can be improved by means of the reinforcing structure formed by the structure reinforcing piece. In other embodiments, the reinforcing structure may also be in other forms; for example, the surface of the connecting member is provided with a rib which is integrally formed with the main body of the connecting member and extended along the extension axis of the tip bending section to form the reinforcing structure.

In addition, in the above embodiments, the service life of the tip bending section of the endoscope can be improved by the connecting member with the reinforcing structure. In other embodiments, the bending fatigue-resistant strength of the connecting member can be improved by changing the material of the connecting member only, thus improving the service life of the tip bending section. For example, when the connecting member and the segment are separately manufactured and fixedly assembled as a whole, the connecting member may be made of materials with higher bending fatigue-resistant strength than that of the segment. In some embodiments, the segment may be made of non-metallic materials, and the connecting member may be made of metal materials with higher bending fatigue-resistant strength than that of the connecting member (such as stainless steel, nickel titanium alloy, etc., which may be in the form of elastic sheet, wire or rope). Alternatively, the segment and the connecting member may be made of non-metallic materials, while the connecting member may made of non-metallic materials with high flexibility (such as PP, PPSU, etc.) or rubber elastomer materials (such as natural rubber (NR), isobutene rubber (IBR), nitrile rubber (NBR)). Alternatively, the segment may be made of non-metallic materials, and the connecting member may be made of high flexibility materials such as fiber rope with bending and resilience performance. For another example, when the connecting member is integrally formed with one of its adjacent segments and is fixedly assembled with another adjacent segment, the bending fatigue-resistant strength of the material used by the connecting member and the segment integrally formed therewith may be higher than that of the material used by the other adjacent segment. In addition, in other embodiments, the material may be changed and the reinforcing structure may be configured at the same time to improve the bending fatigue-resistant strength of the connecting member.

Embodiments of the endoscope in the present disclosure are described as follows.

Please refer to FIG. 1. The endoscope may include a handle 10, an insertion tube 20 and a tip 30. The handle 10 is used for grasping and operating by an operator. The insertion tube 20 is connected to the distal end of the handle 10 for insertion into an organism. The insertion tube 20 includes a tip bending section 21, an extension section 22 and a tip 30. The tip bending section 21 is arranged between the extension section 22 and the tip 30, and can be bent under the control of the handle 10, so as to control the direction of the insertion tube 20 impelled in the organism. The tip 30 is arranged at the distal end of the endoscope for illumination and/or imaging. The tip bending section 21 is the tip bending section 21 mentioned above, which will not be repeated here.

The present disclosure is described and illustrated by means of the above specific examples to only help understand the technical solution of the present disclosure, and not to limit thereto. For those skilled in the art, a number of simple deduction, deformation or replacement can also be made according to the idea of the present disclosure.

## Claims

1. A tip bending section of an endoscope, comprising:
at least two segments; and
a connecting member arranged between two adjacent segments and fixedly connected with the two adjacent segments, the connecting member being capable of being bent and deformed to allow the two adjacent segments to be deflected relative to each other;
a material of the connecting member being different from that of at least one of the two adjacent segments so that bending fatigue-resistant strength of the connecting member is higher than that of the other adjacent segment with different material; and/or, the connecting member having a reinforcing structure for improving the bending fatigue-resistant strength of the connecting member.

2. The tip bending section of the endoscope according to claim 1, further comprising a structure reinforcing piece that forms the reinforcing structure and is fixedly engaged with the connecting member.

3. The tip bending section of the endoscope according to claim 2, wherein the connecting member and the segments are formed integrally; or, the connecting member and the segments are different parts and are fixedly assembled.

4. The tip bending section of the endoscope according to claim 2 or 3, wherein the structure reinforcing piece has a lengthwise direction consistent with an axial direction of the tip bending section.

5. The tip bending section of the endoscope according to claim 2 or 3, wherein the connecting members between every two segments of at least three adjacent segments are arranged along an extension axis of the tip bending section, the at least three adjacent segments form a codirectional bending section with a same deflection direction, and the structure reinforcing piece on the codirectional bending section is arranged in a throughout manner on each segment of the codirectional bending section.

6. The tip bending section of the endoscope according to claim 2 or 3, wherein the structure reinforcing piece is made of metal, fiber or resin.

7. The tip bending section of the endoscope according to claim 6, wherein the structure reinforcing piece is made of one of stainless steel, tungsten, cotton, linen, nylon and PP.

8. The tip bending section of the endoscope according to claim 2 or 3, wherein the structure reinforcing piece is in a form of a single strand or more than two strands.

9. The tip bending section of the endoscope according to claim 2 or 3, wherein the connecting member is provided with a concave portion arranged on an inner wall and/or outer wall of the tip bending section and configured to make a thickness of the connecting member thinner.

10. The tip bending section of the endoscope according to claim 9, wherein the structure reinforcing piece is exposed in the concave portion and fixedly engaged with a bottom wall of the concave portion.

11. The tip bending section of the endoscope according to claim 2 or 3, wherein the structure reinforcing piece and the connecting member are engaged everywhere along an extension axis of the tip bending section.

12. The tip bending section of the endoscope according to claim 11, wherein each segment is an integral injection molded part and the structure reinforcing piece is an injection molded embedded piece wrapped in the integral injection molded part.

13. The tip bending section of the endoscope according to claim 11, wherein the structure reinforcing piece and the segment are bonded and fixed with the connecting member.

14. An endoscope, comprising:
a handle for grasping and operating;
a tip arranged on a terminal of a distal end of the endoscope for illumination and/or imaging; and
an insertion tube connected to a distal end of the handle for inserting into an organism, the insertion tube comprising a tip bending section and an extension section, the tip bending section being arranged between the extension section and the tip, the tip bending section being capable of being bent under a control of the handle so as to be capable of controlling a direction of the insertion tube impelled in the organism;
wherein the tip bending section is the tip bending section according to any of claims 1 to 13.
